# EUROPEAN PATENT SPECIFICATION

(11) **EP 4 169 460 B1**
(45) Date of publication and mention of the grant of the patent: **14.10.2026**
(21) Application number: 21203387.2
(22) Date of filing: 19.10.2021
(51) Int. Cl.: A61B 17/16, A61B 17/17, A61B 17/88

(54) **INSTRUMENT FOR TRANSMITTING TORQUE**
INSTRUMENT ZUR ÜBERTRAGUNG VON DREHMOMENT
INSTRUMENT DE TRANSMISSION DE COUPLE

(43) Date of publication of application: 26.04.2023
(73) Proprietor: Stryker European Operations Limited, Carrigtwohill, Co. Cork T45 HX08 (IE)
(72) Inventor: SIMON, Bernd, 24107 Kiel (DE)
(74) Representative: Röthinger, Rainer

(56) References cited:
- WO-A1-2015/121869
- WO-A1-2015/134750
- WO-A2-2013/033478
- US-A- 4 706 659
- US-A- 5 797 918

## Description

### Technical Field

The present disclosure generally relates to an instrument for transmitting torque.

### Background

In several scenarios, screws need to be fastened or holes need to be drilled. For this purpose, torque-activated tool portions such as screw-driving blades or drill heads are used. The screw-driving blade needs to be turned to fasten a screw, and the drill head needs to be turned to drill a hole.

Commonly used instruments with torque-activated tool portions are rigid and non-flexible. In certain use cases, the screw or the drilling position may be hard or even impossible to reach using such rigid, non-flexible instruments. To address this issue, flexible instruments have been developed that allow for an adjustment of a relative orientation between, for example, a handle of the instrument and the torque-activated tool portion by providing a bendable shaft interconnecting the handle and the torque-activated tool portion.

Current flexible instruments for transmitting torque often suffer from one or more drawbacks. For instance, they may be hard to align relative to a position of interest, and may be difficult to clean.

Flexible surgical instruments are known from documents US 5,797,918 A, WO 2015/121869 A1, US 4 706 659 A, WO 2015/134750 A1 and WO 2013/033478 A2.

### Summary

There is a need for a torque transmitting instrument that solves one or more of the aforementioned or other problems.

Accordingly, an instrument for transmitting torque is provided. The instrument comprises a torque receiving portion at a proximal end of the instrument, and a torque delivering portion at a distal end of the instrument. The instrument further comprises a flexible shaft extending between the torque receiving portion and the torque delivering portion, the flexible shaft comprising torque transmitting elements coupled in series along a longitudinal direction of the shaft such that each of the torque transmitting elements is rotationally fixed and tiltable relative to an adjacent one of the torque transmitting elements. One of the torque transmitting elements comprises a female coupling feature and an adjacent one of the torque transmitting elements (e.g., adjacent to the one of the torque transmitting elements) comprises a male coupling feature matching the female coupling feature. The one of the torque transmitting elements is coupled to the adjacent one of the torque transmitting elements via the female coupling feature and the male coupling feature. The female coupling feature comprises or consists of a notch. The male coupling feature comprises or consists of a protrusion sized to fit into the notch.

The notch may have (e.g., only) a constant width or a narrowing (e.g., decreasing or monotonically decreasing) width starting from an outer surface of the torque transmitting element comprising the notch.

The instrument may be a flexible screwdriver or a flexible drill. The instrument may be configured for use in surgical procedures and thus be referred to as a surgical instrument, although the present disclosure is not limited thereto. When configured for use in surgical procedures, the instrument may be designed to withstand sterilization media.

Each of the torque transmitting elements may comprise a female coupling feature and a male coupling feature (e.g., matching the female coupling feature of the same torque transmitting element). In an alternative implementation, at least one of the torque transmitting elements may comprise two female coupling features or two male coupling features. The torque transmitting elements may be coupled in series by the female coupling features and the male coupling features of the torque transmitting elements.

The notch may have (e.g., only) two side walls. The notch may have only flat or concave side walls. The notch may have a bottom (e.g., a bottom surface). The protrusion may have a free end. The protrusion may have a constant width or a width narrowing (e.g., decreasing or monotonically decreasing) towards the free end thereof. The protrusion may have only flat or only convex side walls. These side walls of the protrusion may be configured to face the side walls of the notch when the protrusion is coupled to the notch, and may therefore also be referred to as coupling side walls of the protrusion. The protrusion may have further side walls in addition to the coupling side walls. The further side walls may be configured to not face the side walls of the notch when the protrusion is coupled to the notch. The further side walls may or may not be flat or convex.

The free end of the protrusion may have a convexly curved end face. The protrusion may thus have a convex height profile, with the maximum height being defined in a center portion of the protrusion and minimum heights being defined at opposite lateral portions of the protrusion. This may allow bending the shaft whilst maintaining a length thereof.

The notch may extend longitudinally along a notch axis. The side walls of the notch may be parallel to the notch axis. The notch axis may be non-parallel, in particular perpendicular, to the longitudinal direction, or longitudinal axis, of the shaft. The notch and the protrusion may be configured such that the adjacent one of the torque transmitting elements, having the protrusion coupled to the notch, can be tilted relative to the notch around an axis non-parallel, in particular orthogonal, to the notch axis.

The notch may have a flat bottom or a concavely curved bottom. A curvature of the concavely curved bottom may correspond to a curvature of the convexly curved end face of the notch. Alternatively, the notch may have a convexly curved bottom. The notch may have a bottom comprising a first bottom section extending from a first end of the notch to a middle of the notch along the notch axis, and a second bottom section extending from the middle of the notch to a second end of the notch along the notch axis. The first bottom section may meet the second bottom section at an angle lower than 180° (e.g., an acute angle, a right angle or an obtuse angle). At least one section chosen from the first bottom section and the second bottom section may be concavely curved.

The protrusion may extend laterally along a protrusion axis. The side walls of the protrusion may be parallel to the protrusion axis. The protrusion axis may be non-parallel, in particular perpendicular, to the longitudinal direction, or longitudinal axis, of the shaft. When a protrusion is coupled to a notch, the protrusion axis of the coupled protrusion may be parallel to or coincide with the notch axis of the coupled notch. At least one axis chosen from the notch axis and the protrusion axis may extend across a radial center of one or more of the torque transmitting elements. At least one axis chosen from the notch axis and the protrusion axis may extend across a radial center of at least element chosen from the one of the torque transmitting elements and the adjacent one of the torque transmitting elements.

The notch of a torque transmitting element may extend non-parallel to the protrusion of the same torque transmitting element. The notch axis of a torque transmitting element may be non-parallel, in particular perpendicular, to the protrusion axis of the same torque transmitting element. The notch of a torque transmitting element may extend essentially orthogonal to the protrusion of the same torque transmitting element. The coupling features of a torque transmitting element may be rotationally offset from one another.

Each of the torque transmitting elements comprises a through-hole. The through hole may be formed in a bottom surface of the notch. The through-hole may extend in the longitudinal direction of the shaft. The through-hole may extend in a center of the torque transmitting element comprising the through-hole. The through-hole may be formed in an end face of the protrusion. The through-hole may end in the bottom surface of the notch and the end face of the protrusion of a torque transmitting element comprising both the notch and the protrusion.

The bottom of the notch and the end face of the protrusion may be configured to allow a tilting between the adjacent one of the torque transmitting elements coupled to the one of the torque transmitting elements, such that the end face of the notch does not slide over the bottom of the notch. This may reduce wear and minimize a change in distance between ends of the through-holes comprised in the one of the torque transmitting element and the adjacent one of the torque transmitting elements.

In the instrument, the torque transmitting elements may be rotationally fixed with respect to rotation around the longitudinal direction of the shaft. The torque transmitting elements may be coupled such that a torque applied around the longitudinal axis of the shaft at one of the torque transmitting elements is transmitted to all others of the torque transmitting elements. The one of the torque transmitting elements may be tiltable relative to the adjacent one of the torque transmitting elements to allow (e.g., a reversible) bending of the flexible shaft. The flexible shaft and the alignment member may be configured such that a relative pose between (e.g., a longitudinal axis of) the torque receiving portion and (e.g., a longitudinal axis of) the torque delivering portion can be reversibly changed. The alignment member may be the only component extending through the through-holes of the torque transmitting elements.

The instrument further comprises an elongate alignment member extending through the through-holes of the torque transmitting elements. The alignment member may be made from a spring-elastic material. The alignment member may be formed from a single piece of material. The spring-elastic material may be a superelastic or pseudoelastic material. The spring-elastic material may be a shape memory material or a shape memory alloy. The spring-elastic material may be a nickel titanium alloy, for example Nitinol.

The alignment member may comprise or consist of a spring wire. The spring wire may have a substantially polygonal or circular cross-section.

In a first variant, the spring wire may not form a coil. In other words, the spring wire may not be rolled, wrapped or wound. The spring wire may be a non-braided wire. The spring wire may extend along the longitudinal direction of the shaft. In its unbiased state, the spring wire may extend in a linear direction, for example at least within each of the through-holes.

In a second variant, the spring wire may form a coil. In this case, the spring wire may be rolled, wrapped or wound. The spring wire may be a braided wire. The coil may extend along the longitudinal direction of the shaft. In its unbiased state, (e.g., a center of) the coil may extend in a linear direction, for example at least within each of the through-holes.

The alignment member, such as the spring wire, may be configured as a bending spring or a leaf spring. The alignment member may be configured to be elastically deformed when the shaft is bent. The alignment member may be configured to, when the shaft is bent, provide a spring force counteracting the bending of the shaft.

The alignment member may be configured to align the torque transmitting elements in a predefined orientation relative to one another. The alignment member may be configured to align the torque transmitting elements such that the longitudinal direction of the shaft extends in a linear direction.

The alignment member may be configured to, when the torque transmitting elements are in the predefined orientation relative to one another, fulfil at least one of the following criteria:
a) the alignment member is in a state of a lowest elastic potential energy;
b) the alignment member is free from a shear force;
c) the alignment member is free from a bending moment;
d) the alignment member is free from a tensile force.

The alignment member may be configured to, when the shaft is bent (e.g., when the torque transmitting elements are not in the predefined orientation relative to one another), be free from a tensile force.

The instrument may comprise at least one recess (e.g., a blind hole) facing the shaft, the at least one recess being formed in at least one component of the instrument chosen from the torque receiving portion and the torque delivering portion. The alignment member may extend into the at least one recess. The alignment member may be configured to align the shaft in a predefined orientation relative to the at least one component.

The alignment member may be configured to, when the shaft is in the predefined orientation relative to the at least one component, fulfil at least one of the following criteria:
a) the alignment member is in a state of a lowest elastic potential energy;
b) the alignment member is free from a shear force;
c) the alignment member is free from a bending moment;
d) the alignment member is free from a tensile force.

The alignment member may be configured to, when the shaft is bent (e.g., when the shaft is not in the predefined orientation relative to the at least one component), be free from a tensile force.

The alignment member may be configured to extend linearly when being in the state of a lowest elastic potential energy.

The instrument may be configured such that the alignment member is translationally fixed along at least one direction chosen from the longitudinal direction of the shaft, a first longitudinal axis of the torque delivering portion and a second longitudinal axis of the torque receiving portion. The alignment member may be attached to one or more components of the instrument chosen from the torque receiving portion, the torque delivering portion and the shaft. The alignment member is attached to both the torque receiving portion and the torque delivering portion. The alignment member may not be attached to one or more, in particular any of the torque transmitting elements.

The torque transmitting elements (e.g., the bottom of the notch and the end face of the protrusion) may be configured to allow a tilting between the adjacent torque transmitting element coupled to the one torque transmitting element, such that the alignment member is not stretched or elongated (e.g., such that the alignment member is only deformed by bending). The torque transmitting elements may be configured such that a change in distance between the ends of the through-holes upon tilting of the torque transmitting elements relative to one another is minimized or prevented. One may say that the torque transmitting elements may be configured to prevent or minimize strain or longitudinal deformation (e.g., elongation or compression) of the alignment member upon bending of the shaft.

A first one of the torque transmitting elements may be coupled to the torque receiving portion such that the first one of the torque transmitting elements is rotationally fixed and tiltable relative to the torque receiving portion. A second of the torque transmitting elements may be coupled to the torque delivering portion such that the second of the torque transmitting elements is rotationally fixed and tiltable relative to the torque delivering portion. The flexible shaft may be coupled to both the torque receiving portion and the torque delivering portion. A torque applied at the torque receiving portion around a longitudinal axis of the torque receiving portion may be transmitted via the torque transmitting elements to rotate the torque delivering portion around a longitudinal axis of the torque delivering portion. A torque applied at the torque receiving portion around a longitudinal axis of the torque receiving portion may result in a (e.g., essentially similar) torque at the torque delivering portion around the longitudinal axis of the torque delivering portion.

The torque delivering portion may comprise a screw-driving tool portion (e.g, a blade or Torx-type feature) adapted for fastening a screw. The instrument may be configured as a screw driver. Alternatively, the torque delivering portion may comprise a drill head adapted for drilling holes. The instrument may be configured as a drill. In still other variants, the instrument may be used for other purposes in which a torque needs to be transmitted.

The instrument may further comprise a handle attached or coupled to the torque receiving portion. The handle may be configured to transmit a torque applied around the longitudinal axis of the torque receiving portion to the torque receiving portion.

### Brief Description of the Drawings

Further details, advantages and aspects of the present disclosure will become apparent from the following embodiments taken in conjunction with the drawings, wherein:
- Fig. 1: shows an embodiment of an instrument in accordance with the present disclosure;
- Fig. 2: shows a longitudinal cross-section of the instrument of Fig. 1;
- Figs. 3a-3b: show different views of a torque transmitting element of the instrument of Fig. 1;
- Figs. 4a-4b: show different views of a torque delivering portion of the instrument of Fig. 1; and
- Fig. 5: shows a torque receiving portion of the instrument of Fig. 1

### Detailed Description

In the following description, exemplary embodiments of an instrument will be explained with reference to the drawings. The same reference numerals will be used to denote the same or similar structural features.

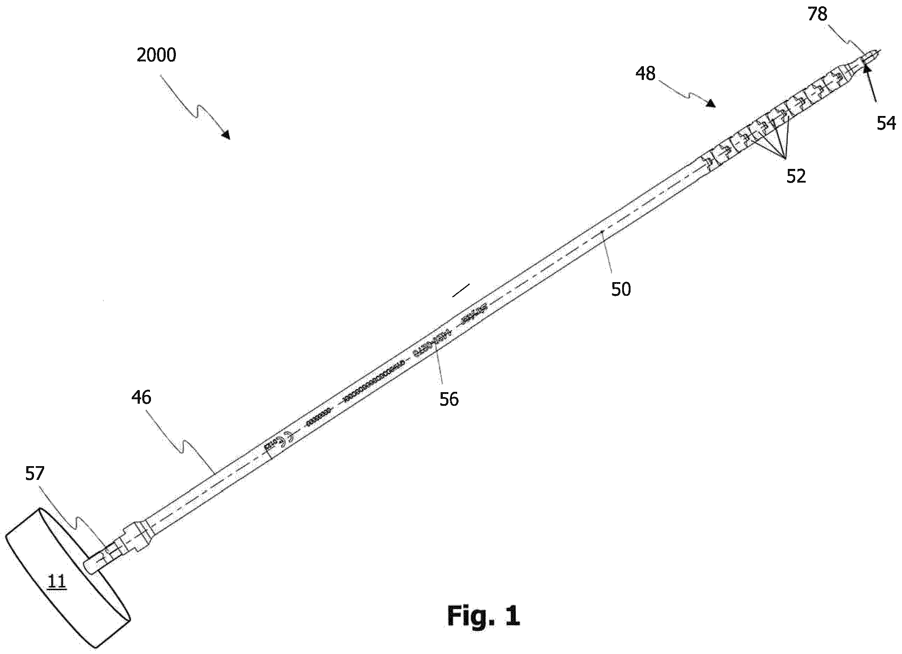

Fig. 1 shows an embodiment of an instrument 2000 in accordance with the present disclosure. The instrument 2000 is adapted for transmitting torque. The instrument 2000 comprises a torque receiving portion 46 at a proximal end of the instrument 2000 and a torque delivering portion 54 at a distal end of the instrument 2000. The instrument 2000 further comprises a flexible shaft 48 extending between the torque receiving portion 46 and the torque delivering portion 54. The flexible shaft 48 comprises a plurality of torque transmitting elements 52. The plurality of torque transmitting elements 52 are coupled in series along a longitudinal direction 50 of the shaft 48 such that each of the torque transmitting elements 52 is rotationally fixed and tiltable relative to an adjacent one of the plurality of torque transmitting elements 52. In some variants, the shaft 48 may comprise between 4 and 40 (e.g., between 10 and 25) torque transmitting elements 52.

One may say that the plurality of torque transmitting elements 52 form a chain along the longitudinal direction 50 of the shaft 48. In other words, the shaft 48 may be formed by the plurality of mutually coupled, or interlinked, torque transmitting elements 52.

The shaft 48 may be flexible in that it is (e.g., reversibly) bendable. That is, an extension of the shaft may be changed from a linear extension to a curved extension and vice versa. Put differently, the shaft 48 may be reversibly bent from a first shape into a second shape from the first shape. The shaft 48 may be bendable by changing a relative orientation between the torque receiving portion 46 and the torque delivering portion 54.

Each of the plurality of torque transmitting elements 52 may be tiltable relative to an adjacent one of the plurality of torque transmitting elements 52 by changing an angle between an axis (e.g., central, longitudinal or rotational axis) of the torque transmitting element 52 relative to an axis (e.g., central, longitudinal or rotational axis) of the adjacent one of the plurality of torque transmitting elements 52. The two axes may be part of or define at least a part of the longitudinal direction 50. By tilting the torque transmitting elements 52 relative to one another, the shape of the shaft 48 may be changed in regard to its extension between the torque receiving portion 46 and the torque delivering portion 54. One may say that by bending the shaft 48, at least some of the plurality of torque transmitting elements 52 are tilted relative to one another.

The plurality of torque transmitting elements 52 may be rotationally fixed relative to one another such that a torque applied around the longitudinal direction 50 of the shaft 48 at one of the plurality of torque transmitting elements 52 is transmitted to all others of the plurality of torque transmitting elements 52. The flexible shaft 48 may be configured such that the shaft can be bent whilst permitting a transmission of torque between the plurality of torque transmitting elements 52 around the longitudinal direction 50 of the shaft 48. The shaft 48 may have an overall cylindrical shape. Each of the plurality of torque transmitting elements 52 may have an essentially cylindrical outer surface forming part of an overall cylindrical outer surface of the shaft 48. Each of the plurality of torque transmitting elements 52 may have the same geometry. Each of the plurality of torque transmitting elements 52 may be rotationally offset from a respective adjacent one of the plurality of torque transmitting elements 52 of the shaft 48.

A first one of the plurality of torque transmitting elements 52 may be coupled to the torque receiving portion 46 such that the first of the plurality of torque transmitting elements 52 is rotationally fixed and tiltable relative to the torque receiving portion 46. A second one of the plurality of torque transmitting elements 52 may be coupled to the torque delivering portion 54 such that the second of the plurality of torque transmitting elements 52 is rotationally fixed and tiltable relative to the torque delivering portion 54.

The flexible shaft 48 may be coupled or rigidly attached to both the torque receiving portion 46 and the torque delivering portion 54. This may ensure that a torque applied at the torque receiving portion 46 around the longitudinal direction 50 is transmitted via the plurality of torque transmitting elements 52 to rotate the torque delivering portion 54 around the longitudinal direction 50. An input torque applied at the torque receiving portion 46 around a longitudinal axis of the torque receiving portion 46 may result in an output torque at the torque delivering portion 54 around the longitudinal axis of the torque delivering portion 54, even if the longitudinal axis of the torque receiving portion 46 is angled or skew relative to the longitudinal axis of the torque delivering portion 54.

The torque delivering portion 54 may comprise a screw-driving tool portion 78 adapted for fastening a screw. In this case, the instrument 2000 may be configured as a screwdriver. The screw driving tool portion 78 may be configured as a hexagonal screw-driving portion. Other forms of the screw-driving portion are also possible (e.g., slot-type blades, cross-type blades, square or star). Alternatively, the torque delivering portion may comprise a drill head (not shown) adapted for drilling holes (i.e., the instrument 2000 may be configured as a drill).

The torque receiving portion 46 may be configured to be coupled to a handle or an electrical actuator such as a motor. As illustrated in the example shown in Fig. 1, the instrument 2000 may further comprise a handle 11 attached or coupled to the torque receiving portion 46. The handle 11 may be configured to transmit a torque applied around a longitudinal axis of the handle 11 to the torque receiving portion 46. The handle 11 may thus be rotationally fixed to the torque receiving portion 46. The longitudinal axis of the handle 11 may coincide with a longitudinal axis of the torque receiving portion 46.

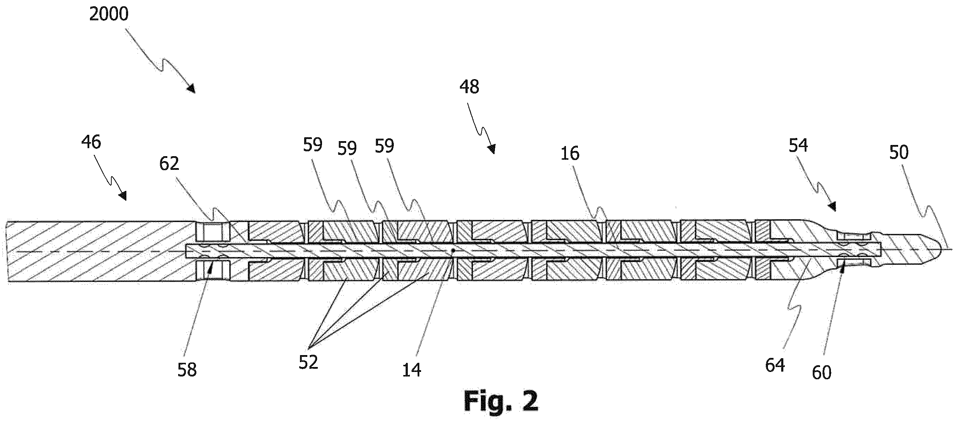

Fig. 2 shows a cross-section of the instrument of Fig. 1 along the longitudinal axis 10. The handle 11 is not illustrated in this figure. As can be seen, each of the torque transmitting elements 52 comprises a through-hole 59. This through-hole 59 may extend along the longitudinal direction 50, for example in a center of the shaft 48. The through-hole 59 may extend in a radial center and along an axial direction of the respective torque transmitting element 52. The through-hole 59 may have a substantially constant diameter (e.g., may be cylindrical).

As illustrated in Fig. 2, the instrument 2000 comprises an elongate alignment member 14 extending through the through-holes 59 of the plurality of torque transmitting elements 52. The alignment member 14 may be made from a spring-elastic material. The alignment member 14 may be the only component extending through the through-holes 59.

The spring-elastic material may be a superelastic or pseudoelastic material. Pseudo-or superelasticity may designate an elastic, reversible response to an applied stress, caused by a phase transformation between an austenitic and martensitic phase of the material. The spring-elastic material may be a shape memory alloy, for example a nickel titanium alloy such as Nitinol.

In the illustrated example, the alignment member 14 consists of a spring wire 16. The spring wire 16 in this example does not form a coil, and is not rolled, wrapped or wound. The spring wire 16 may be a non-braided wire. The spring wire 16 may have a substantially circular or polygonal cross-section. The alignment member 14, in particular the spring wire 16, may have a diameter between 0.8 mm and 3 mm (e.g., ca 1.5 mm to 2 mm). The alignment member 14, in particular the spring wire 16, may have a length between 30 mm and 400 mm (e.g., 70 mm to 200 mm). The spring wire 16 may extend in a linear direction, for example at least within each of the through-holes 59. The spring wire may extend along the longitudinal direction 50 of the shaft 48. It is noted that other variants of the alignment member 14 are also be possible. For instance, the alignment member 14 may form a coil or be a braided wire. The type and configuration of the alignment member 14 may be selected so as to provide the functionality described in the following.

The alignment member 14 may be configured to be elastically deformed when the shaft 48 is bent, and provide a spring force counteracting the bending of the shaft 48. To this end, the alignment member 14 may be configured to align the torque transmitting elements 52 in a predefined orientation relative to one another (e.g., along the longitudinal direction 50 of the shaft 48). This configuration may enable the shaft 48 to take a predefined shape when no bending torque is applied thereto. For example, the alignment member 14 may provide a spring force aligning the plurality of torque transmitting elements 52 so that the shaft 48 extends in a linear direction (i.e., has a straight extension).

The alignment member 14 may be configured to, when the torque transmitting elements 52 are in the predefined orientation relative to one another, fulfil at least one of the following criteria: a) the alignment member is in a state of a lowest elastic potential energy; b) the alignment member is free from a shear force; c) the alignment member is free from a bending moment; the alignment member is free from a tensile force.

The instrument 2000 may comprise a recess 64 facing the shaft 48. The recess 64 may have an essentially cylindrical shape. The recess 64 may be configured as a blind hole. In the example shown in Fig. 2, the recess 64 is formed in the torque delivering portion 54 and extends along a longitudinal direction of the torque delivering portion 54. The recess 64 may be arranged in a radial center of the torque delivering portion 54.

As can be seen in Fig. 2, the torque receiving portion 46 may comprise a further recess 62 configured, in the present variant, as a blind hole. The recess 62 may be directed towards the shaft 48 and may extend along the longitudinal direction 50 of the shaft 48. The recess 62 may be arranged in a radial center of the torque receiving portion 46 and may have an essentially cylindrical shape.

The alignment member 14 may be configured to align the shaft 48 in a predefined orientation relative to at least one component chosen from the torque delivering portion 54 and the torque receiving portion 46. The alignment member 14 in this case may extend at least partially into a through-hole or recess facing the shaft 48 and provided in the respective at least one component. As described above for the plurality of torque transmitting elements 52, this configuration may enable the shaft 48 and the respective at least one component to take a predefined shape when no external bending moment is applied thereon. For example, the alignment member 14 may provide a spring force aligning the plurality of torque transmitting elements 52 and the respective at least one component such that both the shaft 48 and the respective at least one component extend in a linear direction.

The alignment member 14 may be configured to, when the shaft 48 is in the predefined orientation relative to the at least one component, fulfil at least one of the following criteria: a) the alignment member is in a state of a lowest elastic potential energy; b) the alignment member is free from a shear force; c) the alignment member is free from a bending moment; the alignment member is free from a tensile force. The alignment member 14 may be configured to extend linearly (e.g., in a linear direction) when being in the state of a lowest elastic potential energy.

The alignment member 14 may "stiffen" the instrument 2000 such that it extends in an essentially linear direction, while providing for a selected degree of flexibility of the shaft 48. Thus, the instrument 2000 may be more easily handled by a user. For instance, aligning the instrument 2000 using the handle 11 such that the torque delivering portion 54 is at in predefined position may be facilitated.

The instrument 2000 may be configured such that the alignment member 14 is translationally fixed along at least one direction chosen from the longitudinal direction 50 of the shaft 48, the first longitudinal axis and the second longitudinal axis. Optionally, the alignment member 14 may be attached to one or more components of the instrument 2000 chosen from the torque receiving portion 46, the torque delivering portion 54 and the shaft 48. The alignment member 14 (e.g., longitudinal ends thereof) is attached (e.g., forged, soldered, clamped or welded) to the torque receiving portion 46 and the torque delivering portion 54. It may be advantageous if the alignment member 14 extends throughout a through-hole provided in each of the torque receiving portion 46 and the torque delivering portion 54.

Accordingly, the alignment member 14 may not only serve the alignment of the shaft 48, but also ensure that the plurality of torque transmitting elements 52 are restricted in transversal movements along the longitudinal axis of the shaft 48. Also, a loss or decoupling of one component of the instrument 2000 (e.g., the torque delivering portion 54 or one of the plurality of torque transmitting elements 52) may be prevented by the alignment member 14 being attached to the torque receiving portion 46 and the torque delivering portion 54 (e.g., at its longitudinal ends).

In one variant, the alignment member 14 may be arranged such that tensile forces are applied thereon when bending the shaft 48. The alignment member 14 may be configured to be stretched along a longitudinal axis thereof when bending the shaft 48 such that the plurality of torque transmitting elements 52 are misaligned from the predefined relative orientation. This may increase a restoring force provided by the alignment member 14 upon bending of the shaft 48.

In another variant, the alignment member 14 may be arranged such that no tensile forces are applied thereon when bending the shaft 48. This effect may be obtained by appropriately designing the torque transmitting elements such that the shaft can be bent whilst maintaining its length. This may be enabled by the protrusion(s) having a convexly curved end face. The alignment member 14 may thus be configured to not be stretched along a longitudinal axis thereof when bending the shaft 48 such that the plurality of torque transmitting elements 52 are misaligned from the predefined relative orientation. This may improve the lifetime of the alignment member 14.

Referring again to Fig. 1, the torque receiving portion 46 is formed as an (e.g., essentially cylindrical) elongate linear member. An axial length of the torque receiving portion may be longer than an axial length of the shaft 48. Visual indications 56 such as one or more of a manufacturer name, an instrument name or number, certification or warning may be provided on a side or outer surface of the torque receiving portion 46. The torque receiving portion 46 in the illustration of Fig. 1 comprises a coupling portion 57 configured to couple to the handle 11 or an electric actuator (e.g., motor).

With reference to Fig. 2, which shows a longitudinal cross-section of the instrument 2000 of Fig. 1, the torque receiving portion 46 may comprise a first crimp segment 58 fixing (e.g., attaching) the alignment member 14, extending through through-holes 59 of the torque transmitting elements 52, to the torque receiving portion 46.

The torque delivering portion 54 may comprise a second crimp segment 60 fixing (e.g., attaching) the alignment member 14 to the torque delivering portion 54. In this case, the alignment member 14 may not need to extend throughout through-holes in each of the torque receiving portion 46 and the torque delivering portion 54, but may extend into recesses 62, 64 provided in the torque receiving portion 46 and the torque delivering portion 54 and facing the shaft 6, the recesses being optionally formed as blind holes.

The crimp segments 58, 60 may be provided at a longitudinal position of the instrument 2000 corresponding to a longitudinal position of the respective recess 62, 64. Alternative attachment means may be provided instead of the crimp segments 58, 60. For example, the alignment member 14 may be soldered or welded to the torque receiving portion 46 and/or the torque delivering portion 54. The soldering or welding may be applied through a through-hole extending from an exterior of the instrument 2000 to the respective recess 62, 64. In an alternative variant, the alignment member 14 may extend through a through-hole in the torque receiving portion 46 and/or through a through-hole in the torque delivering portion 64 and may be soldered or welded to the respective one of the torque receiving portion 46 and the torque delivering portion 64 at an end of the through-hole facing away from the shaft 48.

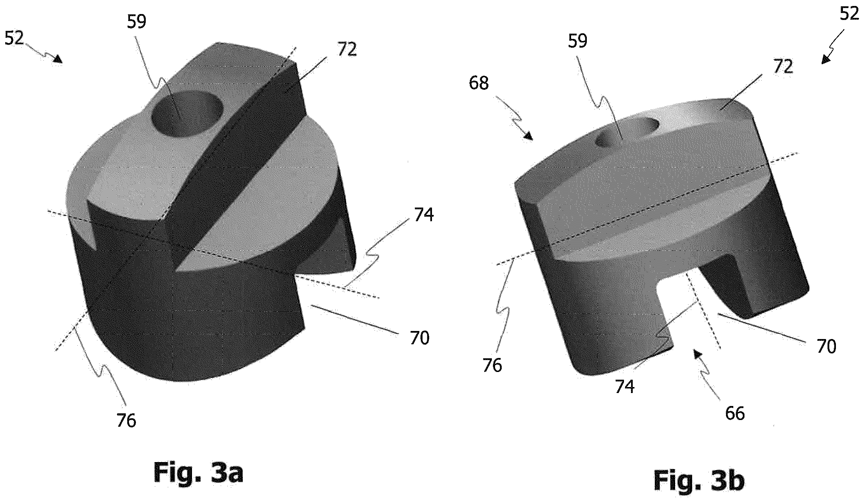

Each of the plurality of torque transmitting elements 52 may have the same geometry and may be rotationally offset from a respective adjacent torque transmitting element 52 of the shaft 48. The torque transmitting elements 52 may have the geometry illustrated in Figs. 3a and 3b.

As can be seen in Figs. 3a and 3b, the torque transmitting element 52 may comprise a female coupling feature 66 and a male coupling feature 68 matching the female coupling feature 66. The plurality of torque transmitting elements 52 may be coupled in series by the female coupling features 66 and the male coupling features 68 of the plurality of torque transmitting elements 52 of the shaft 48.

The female coupling feature 66 comprises or consists of a notch 70. The male coupling feature 68 comprises or consists of a protrusion 72 sized to fit into the notch 70. As illustrated in Figs. 3a and 3b, the notch 70 has a constant width. The notch 70 alternatively tapers from an outer surface of the torque transmitting element 52 toward a bottom surface of the notch 70. The bottom surface of the notch 70 may be flat or concavely curved. In a cross-section across a width of the notch 70, the notch 70 may only have flat or concave walls, and for example have an overall rectangular shape.

The notch 70 may extend non-parallel to the protrusion 72 of the same torque transmitting element 52. The notch 70 may extend orthogonal relative to the protrusion 72 of the same torque transmitting element 52. The notch 70 may extend across a radial center of the torque transmitting element 52. The protrusion 72 may extend longitudinally along a protrusion axis 76. At least one of the notch axis 74 and the protrusion axis 76 may extend across a radial center of the torque transmitting element 52. At least one of the notch axis 74 and the protrusion axis 76 may cut and/or extend essentially perpendicular to the longitudinal axis 50 of the shaft 48. The notch axis 58 may be non-parallel, for example orthogonal, to the protrusion axis 60. The notch 70 may be rotationally offset relative to the protrusion 72 (e.g., around the longitudinal direction 50 of the shaft 52).

In a cross-section across a width of the protrusion 72, the protrusion 72 may only have flat or convex walls and for example have an overall rectangular shape. The protrusion 72 may widen from a longitudinal end of the protrusion (e.g., forming an end of the torque transmitting element 52) towards a base of the protrusion.

As shown in Figs. 3a and 3b, the free end of the protrusion 72 may have a convexly curved end face. The protrusion 72 may thus has a convex height profile, with the maximum height being defined in a center portion of the protrusion 72 and minimum heights being defined at opposite lateral portions of the protrusion 72. The lateral portions of the convexly curved end face may define a maximum tilting angle between two adjacent torque transmitting elements 52 (by coming into abutment against the bottom surface of the adjacent notch 70).

In some variants, the notch 70 may have a concavely formed bottom surface and the protrusion 72 may have a correspondingly formed convex top surface so as to guide a tilting movement of the protrusion 72 of one torque transmitting element 52 in the notch 70 of the adjacent torque transmitting element 52. The notch 70 and the protrusion 72 may be configured such that an adjacent one of the plurality of torque transmitting elements 52, having its protrusion 72 coupled to the notch 70 (e.g., of a certain torque transmitting element 52), can be tilted relative to the notch 70 (e.g., of the certain torque transmitting element 52) around an axis orthogonal to the notch axis 74 of the notch 70 (e.g., of the certain torque transmitting element 52). The adjacent one of the plurality of torque transmitting elements 52 may be tiltable in a plane comprising the notch axis 74 of the notch 70 (e.g., of the certain torque transmitting element 52) and, optionally, the longitudinal direction 50 of the shaft 48). Put differently, the notch 70 and the protrusion 72 may be configured such that an adjacent one of the plurality of torque transmitting elements 52, having its notch 70 coupled to the protrusion 72 (e.g., of a certain torque transmitting element 52), can be tilted relative to the protrusion 72 (e.g., of the certain torque transmitting element 52) around an axis orthogonal to the protrusion axis 76 of the protrusion 72 (e.g., of the certain torque transmitting element 52). The adjacent one of the plurality of torque transmitting elements 52 may be tiltable in a plane comprising the protrusion axis 76 of the protrusion 72 (e.g., of the certain torque transmitting element 52) and, optionally, the longitudinal direction 50 of the shaft 48.

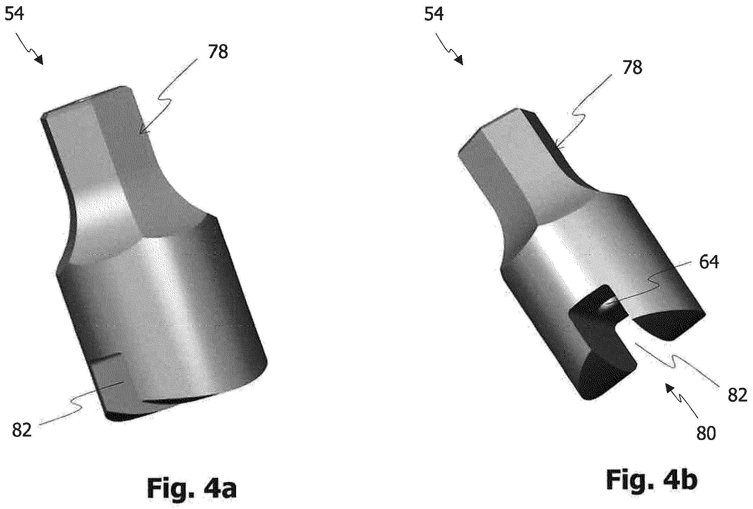

Figs. 4a and 4b show different views of a torque delivering portion 54 of the instrument 2000 of Fig. 1. The torque delivering portion 54 may comprise a coupling feature 80 configured to couple to an adjacent one (e.g., the second) of the plurality of torque transmitting elements 52 of the shaft 48. In this example, the torque delivering portion 54 comprises a notch 82 acting as a female coupling feature for the protrusion 72 of the adjacent one (e.g., the second) of the plurality of torque transmitting elements 52. The notch 82 may be configured similar to the notch 70 described above. It is noted that in an alternative implementation, the coupling feature 80 may be a male coupling feature such as the protrusion 72 described above.

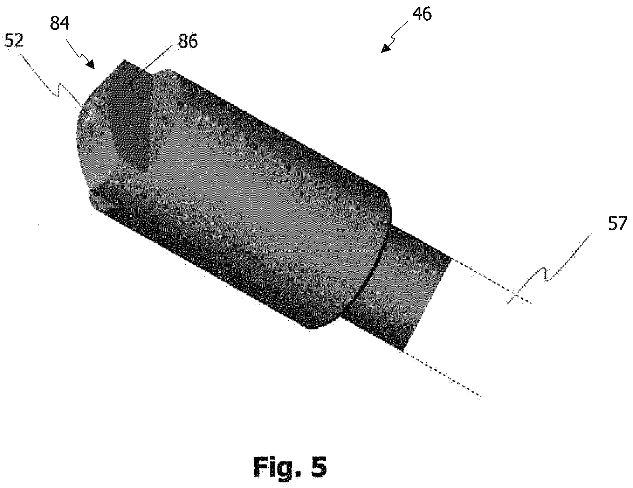

Fig. 5 schematically illustrates a torque receiving portion 46 of the instrument 2000 of Fig. 1. The torque receiving portion 46 may comprise a handle or actuator coupling portion 57 at a longitudinal end thereof (not shown in Fig. 5). The coupling portion 57 may be essentially cylindrical, or prismatic (e.g., hexagonal). At an opposite longitudinal end, the torque receiving portion 46 may comprise a coupling feature 84 configured to couple to an adjacent one (e.g., the first) of the plurality of torque transmitting elements 52 of the shaft 48. In the illustrated example, the torque receiving portion 46 comprises a protrusion 86 configured to couple to the notch 70 of the adjacent one (e.g., the first) of the plurality of torque transmitting elements 52. The protrusion 86 may be configured similar to the protrusion 72 described above and may have a substantially rectangular cross-section. It is noted that in an alternative implementation, the coupling feature 84 may be a female coupling feature such as the notch 70 described above.

Numerous modifications of the instrument 2000 may be possible. In one particular example, the instrument 2000 does not necessarily comprise the alignment member 14. Other variations will be apparent to those skilled in the art. Although numerous advantages have been described herein, the claimed subject matter is not limited thereto. Those skilled in the art will be aware of additional or alternative advantages and technical effects that may be obtained with the subject matter disclosed herein.

## Claims

1. An instrument (2000) for transmitting torque, the instrument comprising:
a torque receiving portion (46) at a proximal end of the instrument (2000);
a torque delivering portion (54) at a distal end of the instrument (2000);
a flexible shaft (48) extending between the torque receiving portion (46) and the torque delivering portion (54), the flexible shaft (48) comprising torque transmitting elements (52) coupled in series along a longitudinal direction (50) of the shaft (48) such that one of the torque transmitting elements (52) is rotationally fixed and tiltable relative to an adjacent one of the torque transmitting elements (52), wherein
one of the torque transmitting elements (52) comprises a female coupling feature (66) and an adjacent one of the torque transmitting elements (52) comprises a male coupling feature (68) matching the female coupling feature (66),
wherein the one of the torque transmitting elements (52) is coupled to the adjacent one of the torque transmitting elements (52) via the female coupling feature (66) and the male coupling feature (68),
wherein the female coupling feature (66) comprises a notch (70) and the male coupling feature (68) comprises a protrusion (72) sized to fit into the notch,
wherein each of the torque transmitting elements (52) comprises a through-hole (59), and wherein the instrument (2000) further comprises an elongate alignment member (14) extending through the through-holes (59) of the torque transmitting elements (52),
wherein the alignment member (14) is attached to both the torque receiving portion (46) and the torque delivering portion (54).

2. The instrument (2000) of claim 1, wherein
the notch (70) has only flat or concave side walls.

3. The instrument (2000) of claim 1 or 2, wherein
at least one of the following conditions holds:
- the notch (70) has a constant width or a narrowing width starting from an outer surface of the torque transmitting element (52) comprising the notch (70); and
- the protrusion (72) has a constant width or a width narrowing towards a distal end thereof.

4. The instrument of any one of claims 1 to 3, wherein
the protrusion (72) has only flat or convex side walls.

5. The instrument of any one of claims 1 to 4, wherein
the notch (70) extends longitudinally along a notch axis (74), wherein the notch (70) and the protrusion (72) are configured such that the adjacent one of the torque transmitting elements (52), having the protrusion (72) coupled to the notch (70), can be tilted relative to the notch (70) around an axis non-parallel, in particular orthogonal, to the notch axis (74).

6. The instrument of claim 5, wherein
the notch axis (70) is non-parallel, in particular perpendicular, to the longitudinal direction (50) of the shaft (48).

7. The instrument of claim 5 or 6, wherein
the protrusion (72) extends longitudinally along a protrusion axis (76) and wherein at least one axis chosen from the notch axis (74) and the protrusion axis (76) extends across a radial center of at least element chosen from the one of the torque transmitting elements (52) and the adjacent one of the torque transmitting elements (52).

8. The instrument of any one of claims 1 to 7, wherein
the notch of a torque transmitting element (52) extends non-parallel to the protrusion (72) of the same torque transmitting element (52).

9. The instrument (2000) of any one of claims 1 to 8, wherein
the alignment member (14) is made from a spring-elastic material.

10. The instrument (2000) of claim 9, wherein
the alignment member (14) comprises or consists of a spring wire.

11. The instrument (2000) of claim 10, wherein
the spring wire is a non-braided wire.

12. The instrument (2000) of any one of claims 9 to 11, wherein
the spring-elastic material is a superelastic or pseudoelastic material.

13. The instrument (2000) of any one of claims 9 to 12, wherein
the alignment member (14) is configured to align the torque transmitting elements (52) in a predefined orientation relative to one another.

14. The instrument (2000) of any one of claims 9 to 13, wherein
the alignment member (14) is configured to, when the torque transmitting elements (52) are in the predefined orientation relative to one another, fulfil at least one of the following criteria:
a) the alignment member (14) is in a state of a lowest elastic potential energy;
b) the alignment member (14) is free from a shear force;
c) the alignment member (14) is free from a bending moment; and
d) the alignment member (14) is free from a tensile force.

15. The instrument (2000) of any one of claims 1 to 14, wherein
the alignment member (14) is translationally fixed along at least one direction chosen from the longitudinal direction (50) of the shaft, a first longitudinal axis of the torque delivering portion (54) and a second longitudinal axis of the torque receiving portion (26), wherein, optionally, the alignment member (14) is attached to the shaft (48).

## Patentansprüche

1. Instrument (2000) zur Übertragung von Drehmoment, wobei das Instrument umfasst:
einen Drehmomentaufnahmeabschnitt (46) an einem proximalen Ende des Instruments (2000);
einen Drehmomentabgabeabschnitt (54) an einem distalen Ende des Instruments (2000);
einen flexiblen Schaft (48), der sich zwischen dem Drehmomentaufnahmeabschnitt (46) und dem Drehmomentabgabeabschnitt (54) erstreckt, wobei der flexible Schaft (48) drehmomentübertragende Elemente (52) umfasst, die entlang einer Längsrichtung (50) des Schafts (48) derart in Reihe gekoppelt sind, dass eines der drehmomentübertragenden Elemente (52) relativ zu einem benachbarten der drehmomentübertragenden Elemente (52) drehfest und neigbar ist, wobei
eines der drehmomentübertragenden Elemente (52) ein weibliches Kopplungsmerkmal (66) umfasst und ein benachbartes der drehmomentübertragenden Elemente (52) ein männliches Kopplungsmerkmal (68) umfasst, das zu dem weiblichen Kopplungsmerkmal (66) passt,
wobei das eine der drehmomentübertragenden Elemente (52) mit dem benachbarten der drehmomentübertragenden Elemente (52) über das weibliche Kopplungsmerkmal (66) und das männliche Kopplungsmerkmal (68) gekoppelt ist,
wobei das weibliche Kopplungsmerkmal (66) eine Kerbe (70) umfasst und das männliche Kopplungsmerkmal (68) einen Vorsprung (72) umfasst, der so bemessen ist, dass er in die Kerbe passt,
wobei jedes der drehmomentübertragenden Elemente (52) ein Durchgangsloch (59) umfasst, und wobei das Instrument (2000) ferner ein längliches Ausrichtungselement (14) umfasst, das sich durch die Durchgangslöcher (59) der drehmomentübertragenden Elemente (52) erstreckt,
wobei das Ausrichtungselement (14) sowohl an dem Drehmomentaufnahmeabschnitt (46) als auch an dem Drehmomentabgabeabschnitt (54) befestigt ist.

2. Instrument (2000) nach Anspruch 1, wobei
die Kerbe (70) nur ebene oder konkave Seitenwände aufweist.

3. Instrument (2000) nach Anspruch 1 oder 2, wobei
mindestens eine der folgenden Bedingungen gilt:
die Kerbe (70) weist eine konstante Breite oder eine sich verengende Breite auf, ausgehend von einer Außenfläche des drehmomentübertragenden Elements (52), das die Kerbe (70) umfasst; und
der Vorsprung (72) weist eine konstante Breite oder eine sich zu seinem distalen Ende hin verengende Breite auf.

4. Instrument nach einem der Ansprüche 1 bis 3, wobei
der Vorsprung (72) nur ebene oder konvexe Seitenwände aufweist.

5. Instrument nach einem der Ansprüche 1 bis 4, wobei
sich die Kerbe (70) in Längsrichtung entlang einer Kerbenachse (74) erstreckt, wobei die Kerbe (70) und der Vorsprung (72) so eingerichtet sind, dass das benachbarte der drehmomentübertragenden Elemente (52), dessen Vorsprung (72) mit der Kerbe (70) gekoppelt ist, relativ zu der Kerbe (70) um eine Achse geneigt werden kann, die nicht parallel, insbesondere orthogonal, zu der Kerbenachse (74) verläuft.

6. Instrument nach Anspruch 5, wobei
die Kerbenachse (74) nicht parallel, insbesondere senkrecht, zu der Längsrichtung (50) des Schafts (48) verläuft.

7. Instrument nach Anspruch 5 oder 6, wobei
sich der Vorsprung (72) in Längsrichtung entlang einer Vorsprungsachse (76) erstreckt und wobei mindestens eine aus der Kerbenachse (74) und der Vorsprungsachse (76) ausgewählte Achse durch eine radiale Mitte von mindestens einem Element verläuft, das aus dem einen der drehmomentübertragenden Elemente (52) und dem benachbarten der drehmomentübertragenden Elemente (52) ausgewählt ist.

8. Instrument nach einem der Ansprüche 1 bis 7, wobei
sich die Kerbe eines drehmomentübertragenden Elements (52) nicht parallel zu dem Vorsprung (72) desselben drehmomentübertragenden Elements (52) erstreckt.

9. Instrument (2000) nach einem der Ansprüche 1 bis 8, wobei
das Ausrichtungselement (14) aus einem federelastischen Material hergestellt ist.

10. Instrument (2000) nach Anspruch 9, wobei
das Ausrichtungselement (14) einen Federdraht umfasst oder aus einem solchen besteht.

11. Instrument (2000) nach Anspruch 10, wobei
der Federdraht ein nicht geflochtener Draht ist.

12. Instrument (2000) nach einem der Ansprüche 9 bis 11, wobei
das federelastische Material ein superelastisches oder pseudoelastisches Material ist.

13. Instrument (2000) nach einem der Ansprüche 9 bis 12, wobei
das Ausrichtungselement (14) so eingerichtet ist, dass es die drehmomentübertragenden Elemente (52) in einer vordefinierten Ausrichtung relativ zueinander ausrichtet.

14. Instrument (2000) nach einem der Ansprüche 9 bis 13, wobei
das Ausrichtungselement (14) so eingerichtet ist, dass es, wenn sich die drehmomentübertragenden Elemente (52) in der vordefinierten Ausrichtung relativ zueinander befinden, mindestens eines der folgenden Kriterien erfüllt:
a) das Ausrichtungselement (14) befindet sich in einem Zustand niedrigster elastischer potenzieller Energie;
b) das Ausrichtungselement (14) ist frei von einer Scherkraft;
c) das Ausrichtungselement (14) ist frei von einem Biegemoment; und
d) das Ausrichtungselement (14) ist frei von einer Zugkraft.

15. Instrument (2000) nach einem der Ansprüche 1 bis 14, wobei
das Ausrichtungselement (14) entlang mindestens einer aus der Längsrichtung (50) des Schafts, einer ersten Längsachse des Drehmomentabgabeabschnitts (54) und einer zweiten Längsachse des Drehmomentaufnahmeabschnitts (46) ausgewählten Richtung translatorisch fixiert ist, wobei das Ausrichtungselement (14) optional an dem Schaft (48) befestigt ist.

## Revendications

1. Instrument (2000) permettant de transmettre un couple, l'instrument comprenant:
une partie de réception de couple (46) située à l'extrémité proximale de l'instrument (2000);
une partie de transmission de couple (54) située à l'extrémité proximale de l'instrument (2000);
un arbre flexible (48) s'étendant entre la partie de réception de couple (46) et la partie de transmission de couple (54), l'arbre flexible (48) comprenant des éléments de transmission de couple (52) couplés en série le long d'une direction longitudinale (50) de l'arbre (48) de telle sorte que l'un des éléments de transmission de couple (52) soit fixe en rotation et inclinable par rapport à un élément adjacent parmi les éléments de transmission de couple (52), dans lequel
l'un des éléments de transmission de couple (52) comporte un élément d'accouplement femelle (66) et un élément adjacent parmi les éléments de transmission de couple (52) comporte un élément d'accouplement mâle (68) s'adaptant à l'élément d'accouplement femelle (66),
dans lequel l'un des éléments de transmission de couple (52) est accouplé à l'élément adjacent parmi les éléments de transmission de couple (52) par l'intermédiaire de l'élément d'accouplement femelle (66) et de l'élément d'accouplement mâle (68),
dans lequel l'élément d'accouplement femelle (66) comprend une encoche (70) et l'élément d'accouplement mâle (68) comprend une saillie (72) dont les dimensions sont adaptées pour s'emboîter dans l'encoche,
dans lequel chacun des éléments de transmission de couple (52) comprend un trou traversant (59), et l'instrument (2000) comprenant en outre un élément d'alignement allongé (14) traversant les trous traversants (59) des éléments de transmission de couple (52),
dans lequel l'élément d'alignement (14) est fixé à la fois à la partie de réception de couple (46) et à la partie de transmission de couple (54).

2. Instrument (2000) selon la revendication 1, dans lequel
l'encoche (70) présente uniquement des parois latérales planes ou concaves.

3. Instrument (2000) selon la revendication 1 ou 2, dans lequel au moins l'une des conditions suivantes est remplie:
- l'encoche (70) présente une largeur constante ou une largeur qui se rétrécit à partir d'une surface extérieure de l'élément de transmission de couple (52) comportant l'encoche (70); et
- la saillie (72) présente une largeur constante ou une largeur qui se rétrécit en direction de son extrémité distale.

4. Instrument selon l'une quelconque des revendications 1 à 3, dans lequel la saillie (72) présente uniquement des parois latérales planes ou convexes.

5. Instrument selon l'une quelconque des revendications 1 à 4, dans lequel
l'encoche (70) s'étend longitudinalement le long d'un axe d'encoche (74), l'encoche (70) et la saillie (72) étant réalisées de telle sorte qu'un élément adjacent parmi les éléments de transmission de couple (52), dont la saillie (72) est couplée à l'encoche (70), puisse être incliné par rapport à l'encoche (70) autour d'un axe non parallèle, en particulier orthogonal, à l'axe d'encoche (74).

6. Instrument selon la revendication 5, dans lequel
l'axe de l'encoche (70) est non parallèle, et notamment perpendiculaire, à la direction longitudinale (50) de l'arbre (48).

7. Instrument selon la revendication 5 ou 6, dans lequel
la saillie (72) s'étend longitudinalement le long d'un axe de saillie (76) et dans lequel au moins un axe choisi parmi l'axe d'encoche (74) et l'axe de saillie (76) s'étend par un centre radial d'au moins un élément choisi parmi l'un des éléments de transmission de couple (52) et l'un élément adjacent parmi les éléments de transmission de couple (52).

8. Instrument selon l'une quelconque des revendications 1 à 7, dans lequel
l'encoche d'un élément de transmission de couple (52) s'étend de manière non parallèle à la saillie (72) du même élément de transmission de couple (52).

9. Instrument (2000) selon l'une quelconque des revendications 1 à 8, dans lequel
l'élément d'alignement (14) est fabriqué dans un matériau élastique à ressort.

10. Instrument (2000) selon la revendication 9, dans lequel
l'élément d'alignement (14) comprend ou est constitué d'un fil à ressort.

11. Instrument (2000) selon la revendication 10, dans lequel le fil à ressort est un fil non tressé.

12. Instrument (2000) selon l'une quelconque des revendications 9 à 11, dans lequel
le matériau élastique à ressort est un matériau superélastique ou pseudoélastique.

13. Instrument (2000) selon l'une quelconque des revendications 9 à 12, dans lequel
l'élément d'alignement (14) est conçu pour aligner les éléments de transmission de couple (52) selon une orientation prédéfinie les uns par rapport aux autres.

14. Instrument (2000) selon l'une quelconque des revendications 9 à 13, dans lequel
l'élément d'alignement (14) est conçu pour, lorsque les éléments de transmission de couple (52) se trouvent dans l'orientation prédéfinie les uns par rapport aux autres, remplir au moins l'un des critères suivants:
a) l'élément d'alignement (14) se trouve dans un état où son énergie potentielle élastique est minime;
b) l'élément d'alignement (14) n'est soumis à aucune force de cisaillement;
c) l'élément d'alignement (14) n'est soumis à aucun moment de flexion; et
d) l'élément d'alignement (14) n'est soumis à aucune force de traction;

15. Instrument (2000) selon l'une quelconque des revendications 1 à 14, dans lequel
l'élément d'alignement (14) est fixe en translation selon au moins une direction choisie parmi la direction longitudinale (50) de l'arbre, un premier axe longitudinal de la partie de transmission de couple (54) et un deuxième axe longitudinal de la partie de réception de couple (26), l'élément d'alignement (14) pouvant, éventuellement, être fixé à l'arbre (48).
